# EUROPEAN PATENT APPLICATION

(11) **EP 0 775 464 A1**
(43) Date of publication of application: **28.05.1997**
(21) Application number: 95203245.6
(22) Date of filing: 27.11.1995
(51) Int. Cl.: A61B 3/12, A61H 5/00

(54) **Apparatus and method to reactivate, normalize, optimize and increase visual functions using retinal reflection measurement**

(71) Applicant: Elderberry Corporation N.V., Curaçao (AN)
(72) Inventor: Bongiovanni, Paolo, 00142 Roma (IT)
(74) Representative: Hooiveld, Arjen Jan Winfried

(57) **Abstract**

An integrated system of stimuli and training also based on a new operating method called Extended Multifunctional Biofeedback (E.M.B.) enables the patient to improve all the physiological functions-particularly visual functions-dependent on the autonomic nervous system. The instrument can also be used as an anomaloscope, as an orthoptoscope, and to determine the critical frequency of central fusion. While not therapeutic, the method can be applied to the restoration of visual abilities impaired by past or ongoing ametropia or eye pathologies, as well as to the treatment of other autonomic nervous system functions. The use of the invention's apparatus has no contraindication.

## Description

The present invention concerns an apparatus and method to reactivate, normalize, optimize, and increase the physiological functions- particularly visual functions-dependent on the autonomic nervous system, also usable as anomaloscope and orthoptoscope and to determine the critical frequency of central fusion.
Such an apparatus and method are known from EP-A-0544631. This known apparatus contains tone feedback means as a perception feedback means for the production of tones having a frequency which is proportional to and/or representative of the readings of the retinal reflection, and contains graphical-numerical visualization means to represent the condition and the variations in the retinal reflection. The disadvantage of the known apparatus is its restricted possibilities in terms of increasing the physiological functions.

### Description

This invention refers to a non-therapeutic instrument and method that can be used to:
- activate, normalize, optimize or increase all physiological functions or abilities dependent on the autonomic nervous system using the E.M.B. (Extended Multifunctional Biofeedback) described below. Merely by way of example, these functions or abilities include cardiac frequency, ability to recover from stress, libido, hunger, thirst, wakefulness/sleep cycles, arterial pressure, etc:
- improve those visual abilities-visual acuity in particular-compromised by ametropia (myopia, hypermetropia, astigmatism, presbyopia, anisometropia);
- augment visual acuity and other visual abilities compromised by existing or past eye pathologies (keratoconus, maculopathy from a variety of origins, retinopathy from a variety of origins, iritis, etc);
- functionally treat binocular anomalies (suppression, convergent/divergent strabismus, disturbing phorias, fixation disparities, amblyopia, etc.);
- increase visual abilities, particularly to treat such learning disabilities as dyslexia, dysgraphia, etc.(improvement of reading comprehension rate, saccadic movements, reading speed, etc.);
- treat neurological problems with visual or ocular importance (nystagmus, photophobia, asthenopia, blepharospasm, sub-atrophy of the optic nerve, etc.);
- apply certain chromotherapeutic therapies, and to quantify dyschromatopsia;
- increase athletic performance by enhancing visual abilities,in all sports where vision plays an important role, such as tennis, football, basketball, volleyball, rugby, baseball, automobile racing, motorcycle racing, handgun or rifle shooting, archery fencing, etc.;

The object of the invention is an instrument and application techniques that are non-invasive, absolutely free of contraindications, and particularly effective, making possible numerous applications of various sensory stimuli E.M.B., chromotherapy, and visual training , also using virtual reality and virtual sound. All or some of these techniques can be performed simultaneously and directly in real space with the goal of involving most of the brain's sensory motor areas at the same time, and the abilities learned during treatment can easily be transferred to everyday life, resulting in shorter application times, improved stability of results, and greater possibilities to increase or restore the functions involved, even where limited by pathologies.

This instrument is marked by its ability to generate particular combinations of infrared radiation at frequencies and intensities suitable for the biochemical activation of the retina. This radiation consists of the emission of an infrared frequency equal to the first harmonic of all colours in the spectrum of visible light. This invention proposes combining the rays with other techniques applicable exclusively with this invention, to reactivate the functions deemed no longer active.

The instrument and its operating methods differ substantially from the apparatus and methods used to date, in that it acts synergically on the functions of the visual system previously not subject to verification for growth, as well as on those functions already subject to verification, but only autonomously. Indeed, this instrument is the only one that makes it possible to subject more than one function to E.M.B. at the same time, with more than one return channel, using different light stimuli and performing increasingly complex functions.

Aside from controlled retinal stimulation, this instrument also carries out various types of monitoring operations at the same time. visual ones (on the retinal reflex, myosis, convergence, accommodation, the accommodation lag, and the AC/A ratio) can be carried out directly with the instrument itself. The other accessory monitoring operations (EEG, EMB, ECG, GSR, etc.) can be acquired using the appropriate electronic port. The instrument can also perform-again at the same time as its other functions-macular massage using red light stimulation at the critical point of central fusion of the receptors (cones) in the area of the fovea. Moreover, the ability to work in conditions of binocularity makes it possible to measure the disparity of fixation and to treat the associated and disturbing phorias, and, thanks to the modulation of the intensity of the mire, to treat suppression and strabismus.

Moreover, the ability to use chromotherapy techniques makes it an ideal coadjutant for certain alternative medicine applications.

The invention can also be used to determine to a high level of accuracy the critical point of central fusion by measuring its inverse critical period of central fusion, and as an anomaloscope to determine possible deficiencies in chromatic perception.

The techniques that can be performed with this instrument are as follows:

### I-Controlled stimulation of retinal activity

Special infrared sources consisting of a number of very low-powered (approximately 2 mW) modulable LED emissions emit several modulable bands at low frequencies in order to have as a frequency the first harmonics of some colours on the spectrum, and are conveyed onto the ocular fundus. These bands are reflected and picked up by a semi-reflective mirror and sent to an optical sensor. Particular combinations of intensity and frequency of the bands emitted correspond to different retinal reflectances. Each patient reacts in his or her own way-increasing the retinal reflectance-to a particular combination of rays, and this can be verified by the variation in the ration between the intensity of reflected light captured by the sensor and the intensity of light emitted. By pinpointing the specific frequency at which this ration is greatest, we will identify-specifically for this patient - the mixture of wavelengths most effective for stimulating the biochemical function of the receptors.

These bands, when mixed together, are collimated by special optic devices and, reflected by the structures of the eye, are picked up by an appropriately coated semi-reflective mirror, which conveys them to an electronic plate where 128 sensors translate them into an electric signal.

The electric signal is then amplified and translated into a variety of return signals (auditory, visual, and tactile) directly or inversely proportional (in height or frequency) to the intensity of the reflected ray.

It is known that retinal reflectance also improves along with improved visual performance and concentration level. For this reason, once the infrared mixture most suitable for stimulating this reflectance is determined, the patient is asked to keep it at levels as high as possible by maintaining the consequent sound, vibration, and light at high levels.

### II-Direct biofeedback of accommodation

The eye's focusing system (accommodation) cannot keep an image perfectly focused on the retina at all times but, as necessary, normally hyperfocalizes or hypofocalizes (negative or positive accommodation) without the individual's noticing the slight "blurring " that takes place. In particular, clinical research has shown that myopic subjects tend to accommodate more than necessary (in front of the reading plane) whilst hypermetropic subjects tend to accommodated less than necessary (behind this plane).

The particular aiming contained in the instrument makes the patient aware of his or her hyper/hypo-accommodation. it consists of thin, discontinuous horizontal lines, thin discontinuous vertical lines, and an intermediate point of fixing so that, when observing the intermediate point of fixing, if accommodation is closer the horizontal lines will appear sharper than the vertical ones, and vice versa in the opposite situation. The discontinuity enables the patient to distinguish better the focus of these aims.

This kind of aiming, which makes the patient immediately and certainly aware of his or her state of accommodation, provides true and direct biofeedback on accommodation to be performed later or, better yet, at the same time as the E.M.B., and the patient can be instructed to change his or her accommodation habit and therefore propensity to hypermetropia or myopia.

### III-Direct biofeedback of convergence

By showing the eye in question, while under normal E.M.B. treatment, the grid mire placed in the optometer (which can be set at any position and therefore at any required level of accommodation within the limits of the optometer itself), and by having the couterlateral eye look at a mire situated in free space, the patient, thanks to the phenomenon of retinal rivalry, perceives the mire inside the grid, in proportion to the position of the visual axes in the absence of fusion.

The patient can then be asked to move the more inside the grid to the right and to the left, thereby teaching him or her to carry out voluntary vergence movements to acquire elasticity of vergence and to counteract adverse esophoria and exophoria.

This technique differs fundamentally from other already known self-styled convergence training techniques carried out in conditions of binocularity by presenting simple mires in artificial (reduced) space, in that it is carried out directly in free space, with the overlapping and fusion of peripheral fields of vision.

### IV-Biofeedback of vegetative functions (electromyography, electroencephalography, electrocardiography)

The machine proposed by this invention can be used as a universal biofeedback machine using an array of peripheral sensors specially constructed for the function to be monitored.

Once can elect for feedback by visual or audio signal or by the above-described tactile response, or by a combination of the three.

### V-Passive foveal stimulation

Patients with low visual acuity both in absolute terms (amblyopia) and with respect to ametropia (lower-than-expected natural visus) generally show a kind of "laziness" of the cones present in the foveal area. The cones, once excited, are not able to discharge within the 22/23 milliseconds typical of well-functioning cones.

The instrument determines the period of inertia (latency) of the receptors, or the critical period of central fusion, and an intermittent flashing square-wave stimulus is sent in the wavelength at which these receptors are most sensitive (635 nanometres) for a period of 2/3 milliseconds more than necessary for them to discharge, so that the patient perceives only a faint flickering.

This continuous charging and discharging of the central receptors in times very close to their maximum performances produces a clear functional-and therefore sensory-improvement that translates into an increased visus.

We preferred to calibrate the instrument in terms of period length (milliseconds) rather than in Hertz (known as an inverse measure to the one chosen) since in this way the necessary clinical increase would vary (the increase in milliseconds is much greater from 15 to 16 Hz than from 60 to 61 Hz).

### VI-Extended Multifunctional Biofeedback

E.M.B. (Extended Multifunctional Biofeedback) is an innovative technique that can be carried out only with the instrument that is the object of this invention.

B.F.B. (of which E.M.B. is an innovation) monitors just one bodily function at a time, giving visual or audio feedback corresponding to the function being monitored. To start with, E.M.B. introduces new and innovative tactile responses (achieved with a system that produces vibrations from 0.5 to 30 Hz that vary in proportion to the function being monitored and that can be perceived by the patient's arm or other part of the body) and visual feedback (by presenting in the parafoveal or peripheral area of the eye being examined or the counterlateral eye a series of low-intensity lights that vary in number, position, intensity, and vibration frequency to represent the function being monitored). But above all, unlike the described B.F.B., E.M.B. envisages simultaneous monitoring of various functions that are interconnected but all dependent on the autonomic nervous system, and a feedback that simultaneously involves more than one sensory channel (for example touch, sight and hearing).

The E.M.B. may be performed either by allocating to each function being monitored a single sensory feedback representative of the latter (for example, a tactile feedback for the E.E.G., a visual return for the G.S.R. and an audio response for the E.M.G.), or by matching the sensory feedbacks taken as a whole to weighed average of the functions being monitored.

This technique, which involves the orthosympathetic and parasympathetic systems, is effective only if all the functions being monitored are dependent on these systems and vary similarly to the variation in the level of activation of these systems.

### VII-Virtual Reality and Virtual Sound techniques

two turrets capable of rotating on the vertical axis around the centre of ocular movements culminate with two semi-reflective mirrors that can be transformed into totally reflective mirrors, with an inclination of 45°, sending the images produced by two small video screens placed within the optometers contained in the turrets.

The reciprocal position of the turrets is changed in order to modify the convergence requirement, while the position of the video screens within the optometers is changed to modify the accommodation requirement.

This system makes it possible to create virtual three-dimensional images, presenting on the two video screens adequate images or stereoscopic mires which the semi-reflective mirrors will make perceivable directly in free space.

Furthermore, mores to compare disparity of fixing, again in free space and with different accommodation requirements, can be presented, thereby making it possible to treat associated and disturbing phorias at any operative distance, working with the E.M.B. techniques afforded by the instrument.

An electronically controlled stereophonic system based on the already known technique of presenting the same sound to both ears at slightly different times (in phase difference mode) will make the audio signal sound as if it came from the perceived virtual image.

Since it is possible to change autonomously both the convergence and the accommodation of the virtual images perceived in real space, acquired bad visual habits can be destabilized, and an enormous number of other visual training techniques can be applied.

### VII-Chromotherapy techniques

The presence within the optical part of an optometer backlit by red, green and blue lights that can be combined in various measures makes it possible to apply numerous chromotherapy techniques.

### IX-Binocular techniques (both eyes looking into free space)

The instrument can rotate 90° to the right and left around the axis passing through the optic centre of the eye being examined thereby making it possible, using a semi-reflective mirror, to apply all the techniques described, even while the eye being examined is observing free space.

This possibility not only allows treatment in monocularity with constant verification of visual acuity, peripheral vision, colour perception, contrast perception, and hence, in general, of the quality of the vision of the eye being treated, but above all permits one to see free space binocularly, in a state of total binocular fusion.

### X-Techniques of vision with alternate occlusion

Two liquid crystal occlusors joined to a horizontal support shaped in such a way that they can be housed in the recess made for this purpose in front of the instrument or to be used independently in front of common eye-glass frames are placed in front of the patient's eyes.

The liquid crystals have the feature of being able to pass almost instantly from a transparent state to opacity.

An electronic control motherboard makes it possible for them to become alternatively transparent for periods pre-established by the trainer. A very rapid alternation of occlusion from one eye to the other makes it possible to pass gradually from a state of monocularity to a state of binocularity (when the alternation of the occlusion is fast enough to not be perceived by the senses), or even to expose the state of transparency of the crystal for a proportionally longer amount of time to one eye than to the other.

Given that under monocular conditions , no adaptations typical of strabismus without diplopia-such as suppression or anomalous retinal correspondence-occur, this technique, used autonomously or jointly with the other techniques possible with this instrument, permits innovative treatment of anomalies of binocular vision.

### XI.Antisuppressive techniques

All the lights introduced to the patient during treatment can be modulated in intensity and/or frequency. Moreover, a sensor measures light in the environment, which can then be modulated as needed.

The suppressing eye can therefore be presented with very bright mires; in the case of the intermittent red light used for passive foveal stimulation, it can be set at the frequency typical of the alpha cerebral rhythm (7-10 Hz), a frequency that tends to inhibit both suppression and anomalous retinal response. The dominant counterlateral eye can be presented with mires having either low illumination or non whatsoever.

In these conditions, obviously, there is no suppression, but by varying the intensity of the mires (diminishing the stimulus to the suppressing eye and increasing it to the dominant eye), we come to the point at which the suppression mechanism intervenes.

In the conditions immediately preceding these limits, retinal rivalry stimulus exercises, especially when carried out jointly with the other techniques illustrated, tend to combat the phenomenon to the point where it ceases altogether.

### XII-Measurement of the critical period of central fusion

This instrument can also be used as a tool to measure the critical period of central fusion, that is, the time that it takes the optic nerve receptors (cones) n the foveal region to charge and discharge.

To determine that amount of time required for the cones to discharge after excitement, it is sufficient to diminish the period of illumination of the stimulator, millisecond by millisecond, until the subject no longer perceives flickering but a continuous light.

The instrument makes it possible to determine this period and therefore the frequency with extreme accuracy, since it is controlled by an internal clock and not by a variable resistance as is found in stimulators on the market (variable resistance is affected by changes in temperature and other environmental variations).

### XIII-Measurement of the ability to identify colours (chromatopsia)

The anomaloscope is an instrument that can be used only to quantify deficiencies in colour perception (dyschromatopsia)

Dyschromatopsia can be classified into protonemaly, deuteranomaly and tritanomaly, depending on which of the fundamental colours, red green or blue, is poorly perceived.

The instrument, which is capable of independently regulating the red, green and blue backlighting of the optometer, can identify each colour with the intensity values of the three colours. To quantify each type of deficiency in colour perception, it is sufficient to present the counterlateral eye with multiple sample colours and to ask the patient to recreate them by regulating the intensity of the optometer's three lights.

To sum up, without having to resort to other techniques, the invention makes it possible to obtain:
- passive foveal stimulation;
- greater stability and learning of one's visual proprioception;
- simultaneous use, by feedback, of the visual, auditory, and tactile channels;
- direct work in free space, without resorting to "transferral of experience";
- direct functional treatment of strabismus;
- resistance to both strabismic and anisometropic suppression;
- subjective and objective real time verification, during the session, of the state of eye convergence;
- direct work to increase the amplitude of accommodation and to vary the AC/A ratio;
- the possibility to create stereoscopic images directly in free space (Virtual Reality).

To conclude, the function being acted on is the visual function in all its complexity and in its characteristic of representing per se the equilibrium of the orthosympathetic and parasympathetic systems, which are direct antagonists only in the visual functions. The patient is subjected not only to specific infrared stimulation but to E.M.B. of convergence, of the AC/A ration and of accommodation (the relationship between accommodation) stimulating binocular vision by constantly exercising retinal rivalry, central vision, and peripheral vision, and at the same time subjecting the patient to another kind of E.M.G. and specific central stimulation to increase the reactivity of the retina and of the corresponding areas of the brain with the presentation at the same time of coloured lights used for chromotherapy techniques.

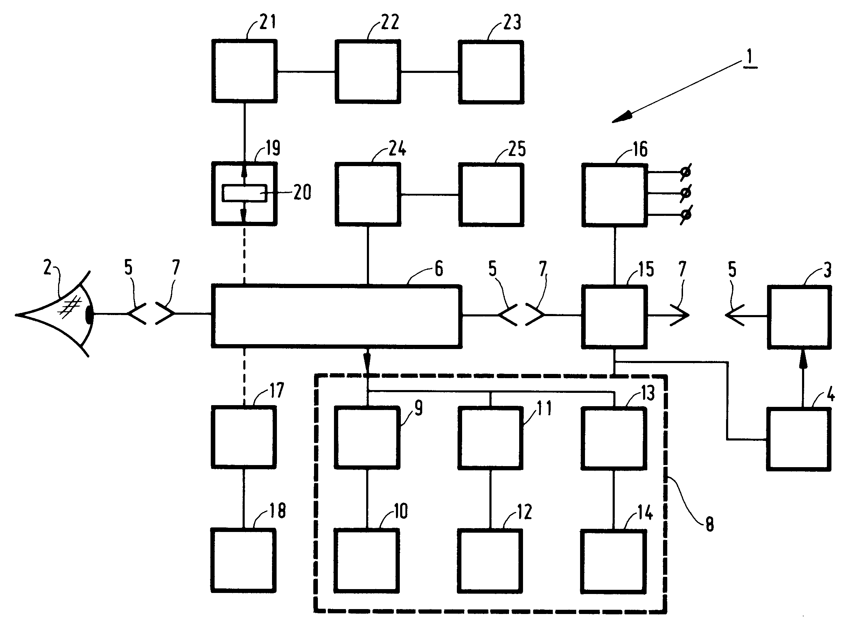

The integration of all these activities gives rise to the considerable results that can be achieved with the apparatus 1 shown in the accompanying figure, particularly in the case of pathologies that reduce visual abilities.

The figure also shows the eye 2. The apparatus 1 comprises an electro magnetic wave source 3 such as preferably an infrared source 3 for transmitting electromagnetic waves to the eye 2. These transmitted electromagnetic waves are modulated by means of modulating control means 4 connected to the source 3. The modulating control means 4 are capable of modulating the transmitted electromagnetic waves either in frequency, in amplitude or in phase, and either continuously or intermittently. The control of these various modulation schemes of the means 4 is controlled by means of control means not shown. Preferable the transmitted infrared wave frequency lies in the range of 800-1400 nm. The transmitted waves 5 pass through optical means 6 and are directed to the retina in the eye 2. The wave 7 reflected by the retina is diverted inter alia to perception feedback means 8. These means 8 contain visual feedback means 9 connected with a display 10; audio feedback means 11 connected with loudspeaker/headphone means 12; tactile feedback means 13, connected with vibrating means 14. All these feedback means generate perception signals in the form of visual, auditive and/or vibrating signals which signal contain a measure for the ratio between the reflected waves 7 and the transmitted wave 5. A part of the reflected waves is also led to analyzing means 15 for determining at an appropriate frequency or frequency band of said modulated waves a maximum of the ratio. The analyzing means 15 is connected to the modulating control means 4 in order to preserve in a preferred embodiment, that the energy of the first harmonic of the visual part of the transmitted waves is substantially kept constant. A control unit 16 is connected with the means 16, which control unit 16 has several inputs for inputting control and process signals, as well as monitor and miscellaneous signals.

In addition the apparatus comprises a fovea stimulator 17, a directing device 18 connected with the stimulator 17, one or more optometers 19 being provided with a monitor 20, an image generator 21 connected with the optometer 19 as well as a virtual sound generator 22 with amplification and loudspeaker means 23 connected therewith. An additional optometer 24 is provided, which is connected with a red green and blue light generator 25.

## Claims

1. An apparatus consisting of an electronic portion and an optoelectronic portion, to produce infrared bands at different and modulable frequencies to stimulate the biochemical function of the retina, which also enables, with the innovative tactile, visual, and auditory feedbacks described below, the applications of E.M.B. to autonomic nervous system functions, with particular regard to visual functions, and which can be used to normalize performances and produce visual, auditory, and tactile sensory stimulation for this normalization, also usable as an orthoptoscope, as a detector of critical frequency of central fusion and of dyschromatopsia, and also including an alternate occlusion device for the treatment-independently of or jointly with E.M.B. - of binocular anomalies, and devices to create images and sounds in Virtual Reality.

2. An optoelectronic apparatus, connected to the electronic control apparatus, consisting of:
- modulable infrared radiation emission apparatus;
- apparatus to detect this radiation reflected by the retinal structure;
- visual feedback apparatus;
- Badal optometer with presentation of moving mires;
- apparatus to emit intermittent red light stimulations at variable intensities and frequencies;
- apparatus to vary the colour and brightness of the background;
- apparatus for the alternating liquid crystal occlusion;
- semi-reflective mirror for the perception of real space at the same time as the application of the specific techniques;
- apparatus to aim the instrument;
- system composed of two screens contained in the Badal optometers to present in semi reflectance types of diagnostic binocular mires or images that can be fused into a virtual image located in real space, with the possibility of changing the patient's accommodation and convergence requirements;
- stereophonic system to simulate the origin of sound stimuli from the virtual image thanks to the phase difference in the sounds perceived by both ears.
These systems are housed in-or in any event, connected to - a suitable container, shaped to enable real space vision by the non-treated eye and, where necessary, also by the eye undergoing E.M.B.

3. An apparatus emitting several continuous or intermittent bands of infrared radiation that can be modulated in frequency without solution of continuity for the entire first harmonic of visible light, placed within the opto-electronic system as described in 2) above. The band of radiations is used to determine which wavelength or combination of wavelengths is most suitable for increasing the efficiency of the retinal tissue in its biochemical function, by evaluating the relationship between the intensity of the ray reflected by the retina structure and that emitted by the instrument. For this measurement, the instrument uses an apparatus that transforms returning infrared light into electric energy and processes this information, determining at which frequency or group of frequencies emitted this ration is highest and therefore corresponds to the maximum increase in visual abilities, and transforming this return ray into a quantity of auditory, tactile, and visual signals, as described below, all functional to the E.M.B. and therefore varying as the return ray varies. Moreover, since we know that retinal reflectance grows with attention, once the most efficient stimulation is determined, the trainer will stimulate the attention of the patient in order to keep the return stimuli high, by further increasing the stimulated activity of the bands of infrared rays.

4. An apparatus to produce visual feedback placed inside the optoelectronic system described under 2) above, consisting of a series of intermittent points of light placed symmetrically on a set of concentric circumferences reflected by a semi-reflective mirror on the same axis as the infrared band described under patent claim 3 above, so that their retinal images fall symmetrically on the perifoveal, parafoveal, or peripheral area.
These lights vary in position and/or number and/or intensity and/or frequency in such a way that the variation is clearly perceived and in line with the variation of the infrared radiation described under point 2) above.

5. An apparatus placed within the optoelectronic system described under point 2) above, consisting of an optometer backlit at varying intensities, containing three mobile mires that can be positioned independently in positions corresponding to any accommodation requirement within the limits of the optometer itself.
The proximal mire consists of thin, continuous or discontinuous horizontal lines. The medial mire consists of a liquid crystal fixing point that can appear and disappear. Lastly, the distal mire consists of thin, continuous or discontinuous vertical lines, so the patient can easily perceive even minimal differences in focusing with respect to the proximal mire. The three mires can also be place in the same position corresponding to the same accommodation requirement, in such a way as to form a grid.

6. An apparatus emitting intermittent square-wave light at varying intensities and frequencies, consisting of two autonomous sources, one inside the optoelectronic system described under point 2) above, and one on the external occlusor, that can move thereon.

7. An apparatus to backlight the optometer placed within the optoelectronic part described under point 2) above, consisting of three lights coloured red, green, and blue respectively, at varying intensities and independently measurable, the combination of which can produce any colour.

8. A rapid alternating occlusion apparatus consisting of two liquid crystals, one for each eye, placed in front of the optoelectronic system described under point 2) above, which can also be used independently of the rest of the apparatus, capable of passing from an opaque to transparent state, entirely or in sectors, alternately or homolaterally with opacity/transparency frequencies ranging from 0.5 to 1,000 Hz.

9. A semi-reflective mirror placed in front of the optoelectronic system described under point 2) above, making it possible to carry out all the functions of the optoelectronic part while the eye being examined observes real space directly and in conditions of binocularity.

10. A pointing apparatus placed within the optoelectronic system described under point 2) above, consisting of a light source projecting a reference mire for the exact pointing of the optoelectronic part.

11. An electronic apparatus with control and command functions connected to the optoelectronic part, including:
- an apparatus to control and command the frequencies of the modulable infrared stimuli
- video for auditory feedback;
- display for auditory feedback;
- display for tactile feedback;
- display for visual feedback;
- E.M.B. sound generator;
- E.M.B. sound generator control;
- apparatus to detect other functions
- mixer/selector apparatus connected to the foregoing, capable of assigning each given feedback (visual, auditory, tactile) independently to each function being monitored, or of mixing data similar to the functions being monitored, producing corresponding and simultaneous variation in these feedbacks
- environmental light measurer;
- automatic timer;
- virtual image and sound control apparatus;
- system for functional electronic self-diagnostics, with flashing indicators corresponding to defective or damaged functions.

12. An apparatus that selects the infrared frequency of the light stimuli.

13. an apparatus for the numerical display of the stimuli described under point 12) above, reflected by the structure of the eye, at the same time as the other E.M.B. functions.

14. An apparatus for the numerical display of the stimuli described under point 12) above, reflected by the structure of the eye, at the same time as the other E.M.B. functions.

15. An apparatus for the generation of continuous or rhythmic feedback sounds, which can be varied in height an/or frequency with variations representing the stimulus described in point 12) above reflected by the structure of the eye, at the same time as the other E.M.B. functions.

16. An apparatus to control the vibration generator. This generator is placed in contact with the patient's body, on order that he or she may easily perceive the variations representing the stimulus described in point 12) above, reflected by the structure of the eye, at the same time as the other E.M.B. functions.

17. An apparatus for the simultaneous monitoring of the functions that depend on the autonomic nervous system such as, for example, visual potentials evoked, cerebral rhythms, cardiac rhythm, respiratory rhythm, galvanic conductivity of the skin, muscle tension etc.
Variations in these functions, translated by already known apparatus into electrical signal variations consistent with and proportional to these, reach the electronic apparatus described under point 11) above, where they are associated by a mixer/selector apparatus with multiple multisensorial feedbacks, or each connected to a specific function being monitored, or are, taken together, consistent with and representative of the set of functions being monitored.

18. An apparatus to be used for improving one or more physiological functions of a living being such as a person, the apparatus comprising:
- an electromagnetic wave source;
- optical means for directing said electromagnetic waves to the retina of the eye of the person and passing on the waves reflected from the retina;
- perception feed back means coupled to the optical means for providing to said person at least a perception signal derived from said reflected waves, characterized in that
- the apparatus further comprised modulating control means coupled to said electromagnetic wave source for modulating said electromagnetic waves directed to said person.

19. Apparatus according to claim 18, characterized in that the modulating control means are equipped to modulate said electromagnetic waves in frequency, or amplitude or phase.

20. Apparatus according to one of the claims 18,19, characterized in that the modulating control means are capable of being operated continuously or intermittently.

21. Apparatus according to one of the claims 18-20, characterized in that modulating by the modulating control means takes place in such a way that the energy of the first harmonic of the visual part of the electromagnetic waves of the wave source is substantially kept constant.

22. Apparatus according to one of the claims 18-21, characterized in that the apparatus comprises analysing means for determining at an appropriate frequency or frequency band of said modulated waves a maximum of the ratio between the reflected waves and the waves transmitted by the electromagnetic wave source.

23. Apparatus according to one of the claims 18-22, characterized in that the perception feedback means contain audio feedback means and/or visual feedback means and/or tactile feedback means.

24. Apparatus according to one of the claims 18-23, characterized in that the frequency of the modulatable electromagnetic waves directed to the person lie in the infrared range.

25. Apparatus according to one of the claims 18-24, characterized in that the frequency is in the range of 800-1400 nm.
